# EUROPEAN PATENT SPECIFICATION

(11) **EP 4 455 647 B1**
(45) Date of publication and mention of the grant of the patent: **02.04.2025**
(21) Application number: 23382387.1
(22) Date of filing: 26.04.2023
(51) Int. Cl.: G01N 22/00, G01N 33/00

(54) **SENSOR AND SENSOR SYSTEM FOR GAS DETECTION**
SENSOR UND SENSORSYSTEM ZUR GASDETEKTION
CAPTEUR ET SYSTÈME DE CAPTEUR POUR DÉTECTION DE GAZ

(43) Date of publication of application: 30.10.2024
(73) Proprietor: CENTRE TECNOLOGIC DE TELECOMUNICACIONS DE CATALUNYA (CTTC), 08860 Castelldefels (ES)
(72) Inventor: LLAMAS, Ignacio, 08860 Castelldefels (ES); BRITO, Zabdiel, 08860 Castelldefels (ES)
(74) Representative: Mohammadian, Dario

(56) References cited:
- US-A1- 2009 312 954
- US-A1- 2017 138 869
- BOUVET M ET AL: "Molecular Semiconductor-Based Gas Sensors", ENCYCLOPEDIA OF SENSORS, X, XX, vol. 6, 1 January 2006 (2006-01-01), pages 227 - 269, XP009094472

## Description

### TECHNICAL FIELD

The present invention relates generally to the field of sensors, and in particular, to an improved small, low-cost planar wireless sensor for sensing gases, together with corresponding system.

### BACKGROUND OF THE INVENTION

Stand-off detection of toxic chemical compounds, such as nerve agent gases, is critical for security. Contaminated zone surveillance can be performed using unmanned ground vehicles carrying a sensor tag. Numerous proposals have been made for the design and implementation of sensors to detect chemical agents used in chemical warfare. Given the importance and interest in detecting and monitoring areas contaminated with chemical weapons, there is a great variety of detection techniques, each with its limitations, advantages, and disadvantages.

Traditional implementations involving the use of infrared lasers are large, heavy, and costly. Another solution is the use of Raman-type spectroscopy, however, this technique is not suitable for realistic environments due to its low reliability. Colorimetric sensors are very practical and printed on paper, however they are generally limited to detect liquids, and often produce false alarms. These sensors have the potential of detecting gases in tubes, however, they result in large sensors wherein an air pump is required, and they have a delayed response time. Ion mobility spectrometers can be used to detect chemical warfare agents with portable equipment. However, their operation requires a radioactive material and is an expensive solution. They are often combined with gas chromatographs; however, these are large, expensive and require trained personnel to use the equipment. The same happens when using flame photometry, which uses polymers on acoustic surface wave circuits, however, issues of detection sensitivity and low robustness are appreciated in the designs. The micro-resistor arrays made with metal oxide semiconductor offer advantages such as reduced size, low-cost and fast response, however, these designs lack sensitivity. Also, wireless hazard badges to detect nerve-agent simulants are known which use single walled carbon nanotube-based chemi-resistive dosimetric materials however the production is extremely complex and the solution not cost effective. RFID chipless sensors based on split ring resonators implemented using inkjet printing are known, however, they use a horn antenna, which is bulky, and exhibits low sensitivity, and therefore are not suitable for low-cost implementation on unmanned vehicles. Publication US-A-2017/0138869 relates to a sensor system for detecting gases by RF/microwave by taking readings from two sensors, each sensor comprising a monomolecular layer of graphene which is complex in structure and construction. Publication US-A-2009/312954 refers to a sensor system for detecting gases based on a buried heater configuration on a thin film (10-100 nm) dense microstructure sensor configuration operating at very low frequencies (maximum 0.001 GHz) with careful control of variable operational temperature settings.

Therefore, a need exists to effectively solve the abovementioned problems.

### SUMMARY

It is therefore an object of the present invention to provide solutions to the above-mentioned problems. The invention is defined in the claims. In particular, it is an object of the invention to provide a novel planar sensing apparatus which is small and substantially flat whilst at the same time exhibiting high sensitivity whilst detecting gases.

Therefore, it is an object of the present invention to provide an improved sensor for gas detection.

It is another object of the present invention to provide a system for sensing gases comprising the improved sensor.

### BRIEF DESCRIPTION OF THE DRAWING(S)

The features and advantages of the present invention will become more apparent from the detailed description set forth below when taken in conjunction with the drawings in which like reference characters identify corresponding elements in the different drawings. Corresponding elements may also be referenced using different characters.
**FIG. 1** depicts a sensor tag layer configuration according to one aspect.
**FIG. 2** depicts a sensor tag layer configuration according to another aspect.
**FIG. 3** depicts a sensor tag layer simplified configuration with a compact stack-up arrangement.
**FIG. 4** depicts a transmission line sensor configuration according to one aspect.
**FIG. 5** depicts a transmission line sensor configuration according to another aspect.
**FIG. 6** depicts a microwave circuit sensor configuration according to another aspect.
**FIG. 7** depicts a microwave circuit sensor configuration according to another aspect.
**FIG. 8** depicts a resonator-based sensor model according to another aspect.
**FIG. 9** depicts a resonator-based sensor model according to another aspect.
**FIG. 10** depicts a system for the remote detection of gases.
**FIG. 11** depicts 4 different sensor tags.
**FIG. 12** depicts typical transmission changes when a sensor transmission line is exposed to low gas concentrations.

### DETAILED DESCRIPTION OF THE INVENTION

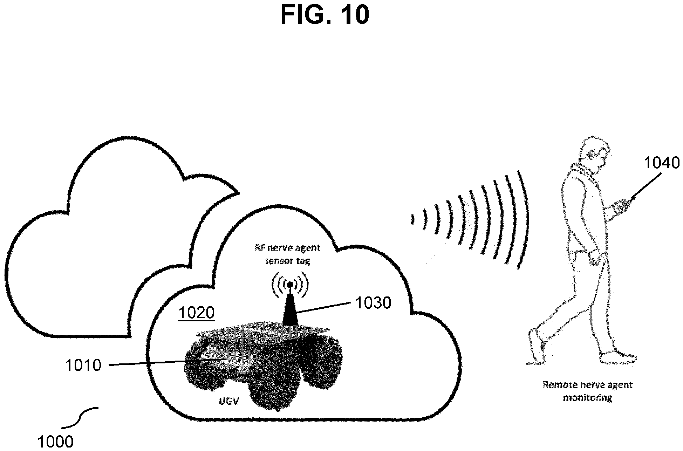

**FIG. 10** depicts an example system 1000 for the remote detection of gases according to one aspect. Typically, an unmanned vehicle 1010 is sent into a contaminated area 1020 to detect potentially harmful gases for human beings for remote nerve agent detection and site monitoring. The vehicle, which can be a ground vehicle UGV, but may also be airborne, carries a sensing apparatus 1030, or sensor, which comprises at least one sensor module configured with at least one electric circuit. A processing module is configured to cause an electric current to flow through the sensor circuit of the sensor module and to determine sensor data indicating the presence of gas. The processing module may optionally comprise a radio frequency emitter for irradiating radio frequency waves onto the sensor module for operation. The sensor may optionally also comprise a communications module to transmit the sensor data to a remote electronic device 1040. The electronic device is normally carried by a human operator in a safe zone away from the contaminated zone, who can monitor the situation and take corresponding decisions without being subjected to gas exposure. Alternatively, the RF emitter and/or communications module could be configured external to the sensor.

In order to safely detect nerve agent gases, such as sarin, a chemically equivalent compound is used. In this case, dimethyl methylphosphonate, DMMP, has been used to test the sensor of the invention. The wireless sensor tag uses microwave signals to perform the detection of nerve agents, such as DMMP, a chemical compound used to produce chemical weapons, it is common practice to develop nerve agent sensors by detecting the DMMP compound. The description will mainly use DMMP as an example gas, however the invention is not limited to the detection of DMMP or sarin, and can generally detect any gas allowed by the particular material and layer configuration of the sensor tag.

The sensor has been designed to operate generally in radio frequencies, RF, however, due to its intrinsic properties, higher frequency microwaves are preferred as they result in smaller devices. Although the objective is to produce low-cost sensor tags using sub 6 GHz frequencies, measurements up to 67 GHz and beyond are possible where the detection of DMMP can be achieved with high sensitivity. In one aspect, the main sensor tags and tag reader use sub 6 GHz frequencies, where microwave components are very low-cost and easy to integrate with the proposed planar sensor tags as surface mounted components. The microwave circuits are aimed to operate below 6 GHz compatible with radio frequency identification standards, thus the microwave signals are used for sensing DMMP and transmitting the signal wirelessly to a small and low weight tag receiver. The description will mainly focus on microwave circuits operating in the microwave range of frequencies, however the invention is not limited to operation specifically in this range of frequencies.

The term "sensor tag" herein is used to refer to a planar device comprising stacked elements necessary for sensing, and will be referred to interchangeably also as sensor module. The sensor tags can be fabricated on low-cost microwave laminates, glass, quartz, or flexible plastic materials as the substrate. The choice of the combination of materials for the different layers enables the advantages described. The noble metal palladium, graphene, or metal oxide layers, are used as active layers for the detection of DMMP. The layers are deposited on the substrate, and different aspects of the invention are directed to different combination of layer numbers, functionality as well as materials. The invention enables a substantially planar configuration, very apt for its integration into other electronic devices as it occupies little volume and has a large surface area of contact.

The active sensor layer can be used embedded in classical microwave circuits to form sensors, such as interferometer circuits, resonant circuits, antennas and filters, implementations supporting surface waves or spoof surface plasmon propagation and simple lumped elements such as interdigital capacitors and meander inductors. Microwave laminates can be used as substrates, which contain thick low loss copper lines, the sensor active layer can be sputtered, e-beam evaporated, or atomic layer deposited using a mask defining the sensor active region over the low loss copper lines and substrate to form sensors. The sensor tags are made with traditional microwave circuits, such as transmission lines, filters, resonators, inductors, capacitors, or antennas. Sensor tag designs incorporate stacked layers of materials to form the microwave circuits, the stacked layers allow gas detection, since their electrical properties change with the presence of the gas. The invention is not directed to the process of manufacturing the sensor tag, for which standard state of the art manufacturing processes are used, which are readily available to the person skilled in the art for reference.

Material electric property changes, such as resistivity, conductivity or permittivity, are used to identify the presence of gas and can be obtained by observing the response of the microwave circuits using microwave signal response analysis. To this end, the processing module is configured to cause an electric current to flow throughout the electrical circuits of the sensor module, in particular, its active layers. The processing module is configured to determine a property as a function of the electric current when in a neutral environment, that is, in the presence of an environment without the gas to be determined. Once the sensor is placed in the contaminated environment, that is, the environment with the gas to be determined, the signal flowing through the circuit is affected by the change in a property (such as electric resistivity, conductivity, or permittivity) of the stacked materials under the presence of the gas. The processing module is configured to detect this change in property and determine the presence of the gas.

Specific parts of the microwave circuit used as sensor are designed to contain the active material so as to perform the sensing as a function of loss changes, resonant frequency, or phase variations to the microwave signal. The microwave signal can be transmitted remotely to the sensor tag using an embedded chip on the sensor, or the sensor can be passive and chip-less, thereby allowing the radio frequency impingement from alternative sensor-external RF sources. Which change of property is used to determine the presence of gas can be a matter of design choice, as it is the features of the invention which enable the highly sensitive detection of property changes in the presence of gas. The detection using microwave signals and circuits is done in the form of device insertion or reflection loss, phase shift, including resonant frequency variation when the proposed sensor tags are exposed to the gas, even at low concentrations. The microwave signals flowing through the sensor tags are used for nerve agent detection and for wireless sensor data transmission, simultaneously, thus allowing detection at a distance using a handheld tag reader, or reading device. The sensor is calibrated in an environment free of those gases which are to be detected and its basic parameters measured (such as loss changes, and/or resonant frequency, and/or phase variations, and/or resistivity, and/or conductivity and/or permittivity, or others).

In one aspect, the sensor module is implemented as an active module. In the standby state, it has an electric signal in the microwave frequency range running through its active layer, as fed by the processing module. The processing module is configured to continuously determine the basic parameters and any changes therein, and to make the data available, either by storing or transmitting to an external electronic device. Once in the presence of gas, the change in basic parameters is thereby detected.

In another aspect, the sensor module is implemented as a passive module. In the standby state, it has no electric signal running through its active layer. Once sensing is to be performed, a microwave frequency wave is irradiated onto the sensor, which converts the waves into a corresponding microwave frequency electric signal flowing through its active layer. The processing module is configured to continuously determine the basic parameters and any changes therein, and to make the data available, either by storing or transmitting to an external electronic device. Once in the presence of gas, the change in basic parameters is thereby detected. In this configuration, the calibration step can be optionally be performed before the sensor is present in the environment containing the gas to be detected.

Based on the changes caused by the presence of gas, the processing module is configured to indicate the presence of gas, and/or which type of gas, and/or its concentration level. This information is either stored locally or transmitted externally to an external electronic device, or reader. The overall dimensions of the electronic reading device is approximately 10 cm long, 5 cm wide and 2 cm tall, weighing less than 100 grams approximately. The electronic reading device proposed includes a small rechargeable battery if configured as a wireless electronic device. The invention is not limited to these dimensions, as advances in manufacturing technology will enable even smaller devices.

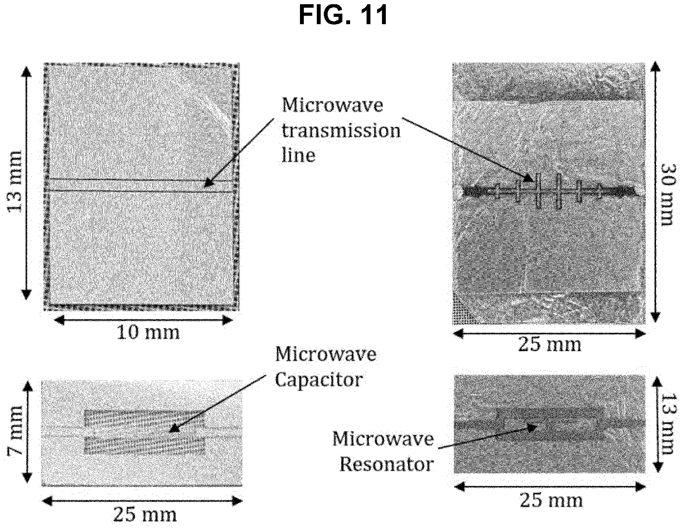

**FIG. 11** depicts 4 different sensor tags and their dimensions. Each one is configured as a planar RF circuit with a different configuration of layers and conducting line patterns. The layers are numbered starting from the bottom substrate upwards. The above tags correspond to microwave transmission lines whereas the bottom left corresponds to a microwave capacitor and the bottom right to a microwave resonator. The sensor tags are all configured starting from a substrate of quartz which is a low loss substrate. The different layers are constructed from specifically selected materials, such as zinc oxide, titanium oxide or palladium, which allow the detection of toxic gases due to intrinsic electrical property change when exposed to the gas.

In this process, it was discovered that producing thick films, for instance depositing a 500-800 nm thick palladium film over a gold layer of 40 nm, is necessary to optimize performance due to the skin depth effect when operating at microwave frequencies. Sensor design considers optimizing such layers for maximum sensitivity. However, it has been shown that a high residual stress remains. Residual stress (or residual strain) is the stress that remains in a solid material after the cause that produces it has ceased, and is an undesirable effect as it affects the physical integrity of the sensor, causing layer separation and sensor malfunction. In order to minimize this drawback, a number of different layer combinations have been developed.

It was discovered that the direct contact of thin palladium layers onto the adhesion layer caused severe energy losses. The energy losses are due to the skin depth effect, where very thin metals exhibit high loss when compared to the skin depth at the frequency of operation. Hence, it was decided to add an intermediate gold layer in order to minimize the losses. Alternatively, to gold, it is also possible to use silver or copper to reduce the losses.

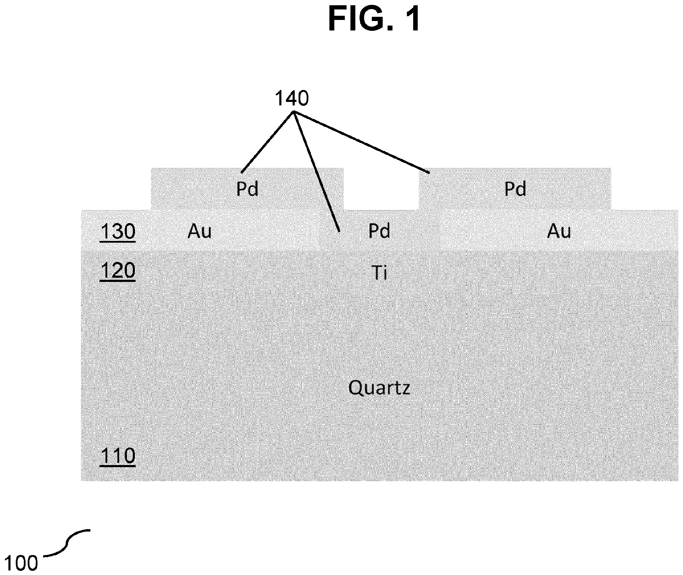

In one aspect, as depicted in **FIG. 1****,** a sensor tag 100, or sensor module, is configured wherein the first layer is a quartz SiO₂ substrate 110 with a third layer of gold Au 130 deposited above. A second layer of titanium Ti 120, as a thin seed layer, is used as an adhesion layer to maximise the adhesion of the gold deposition on the quartz substrate. Finally, the sensing layer is deposited as a fourth layer 140 of palladium Pd. In this configuration overlapping metal sections ensure good electrical contact and larger sensor active area, for example, as can be seen, the Pd sensing layer overlaps the gold layer allowing a larger sensor active area. Alternatively, to titanium, it is also possible to use chromium Cr as an adhesion layer material. In one configuration, typical layer thicknesses are 500-1000 µm for layer 1 (525 µm in one example implementation), 5-10 nm for layer 2 (5 nm in one example implementation), layer 3 ranges from 0.5 µm - 3 µm (0.5 µm in one example implementation), and layer 4 ranges from 40 nm to 3-5 µm (40 nm in one example implementation).

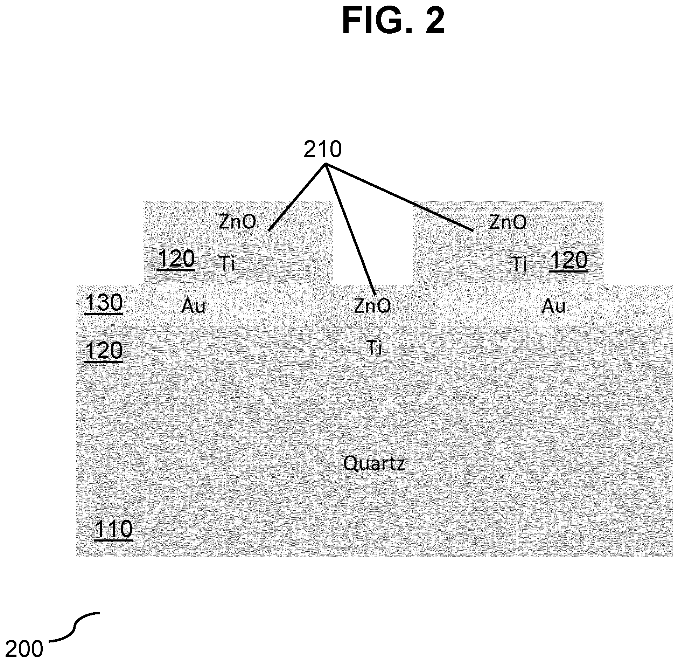

As an alternative to using palladium as the active sensing layer, it is also possible to use a thin layer (thickness between 10-40 nm) or thick layer (0.5-3 µm) of a metal oxide to increase the sensitivity to gas detection, as depicted in the sensor tag 200 of **FIG. 2****.** Metal oxides are used to detect the presence of gases by measuring the resistance change of the metal oxide, and they are widely used due to their excellent sensing properties, abundance, and ease of fabrication. In this aspect, one of zinc oxide ZnO, titanium oxide TiO₂, tin oxide SnO₂, copper oxide CuO, or tungsten oxide WO₃, are used as the sensor active layer.

The deposition of thick layers 210 (thickness between 3-5 µm) minimizes sensitivity, and lowers losses, however the drawback is that the adhesion quality decreases the thicker the top layer gets, and residual stress builds up. Hence, another layer of titanium 120 is added in between the top layer and the gold layer 3.

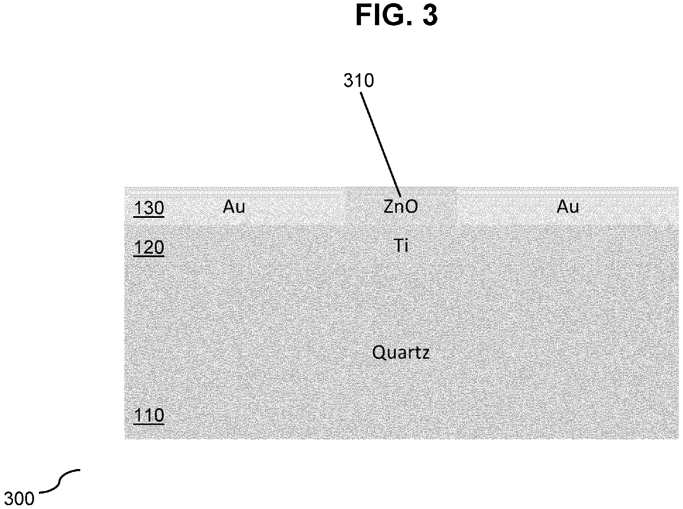

**FIG. 3** depicts a sensor tag 300 simplified configuration with a compact stack-up configuration using a metal oxide sensing layer 310 (one of ZnO, TiO₂, SnO₂, CuO, or WO₃) with a chromium Cr or titanium Ti seed layer, and gold Au, silver Ag or copper Cu to attain low loss sensor design (0.5 - 3 µm thick). This simplified topology allows having a confined area of the sensitive layer while the rest of the structure is made of a highly conductive metal layer.

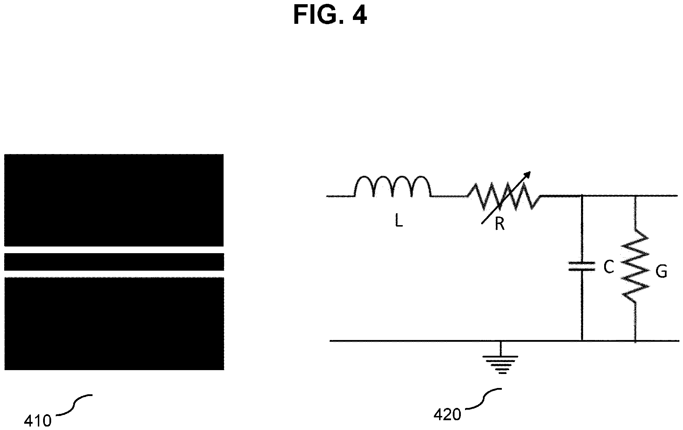

Each one of these different material stack layer combinations can be used in diverse microwave circuit layouts or topologies, for example transmission lines, resonators, capacitors, inductors, interferometers or antennas. In one aspect 410 of **FIG. 4****,** the coplanar waveguide CPW line is configured as a resistive sensor. Gas molecule interaction with the sensor active layer modifies the conductivity of the selective Pd metal or metal oxides (ZnO, TiO₂, SnO₂, CuO, or WO₃). The equivalent electric circuit model 420 of the CPW line is used to estimate sensor response wherein microwave circuit areas containing the active materials mainly act as variable resistors. This model contemplates conductivity losses as a resistance R, so that the presence of gas modifies the value of R. The resistivity is reduced in the presence of gas. The CPW line has both signal and ground planes in the same sensitive layer, this topology presents a high sensitivity since the area of the sensor active layer is large and where both signal line and ground plane are coated with the active layer.

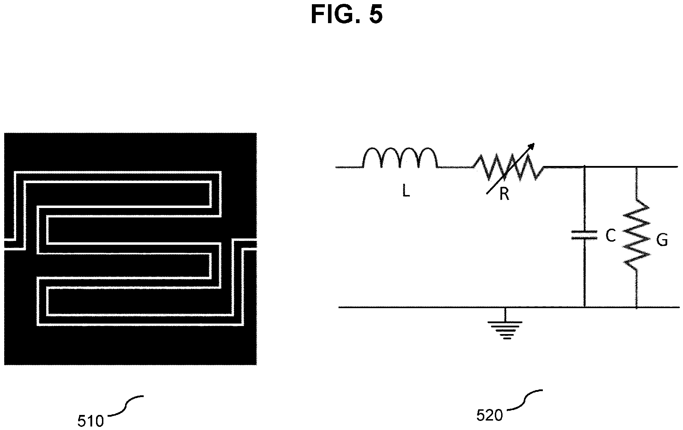

**FIG. 5** depicts a resistive sensor configured as a delay line. The conducting strip is a long transmission line made with the active layer, Pd metal or metal oxides (ZnO, TiO₂, SnO₂, CuO, or WO₃). Due to the increased folded transmission line length, the change of resistance is higher since the microwave signal has to travel a larger distance and thus resistivity change is higher when exposed to the gas, so the overall sensor sensitivity is increased. This topology combines the use of CPW and a long transmission line. Such a combination allows the resistivity variation of the sensitive layer to be higher and therefore increase the sensitivity of the sensor. The equivalent electric circuit model 520 of the delay line is used to design and estimate sensor response.

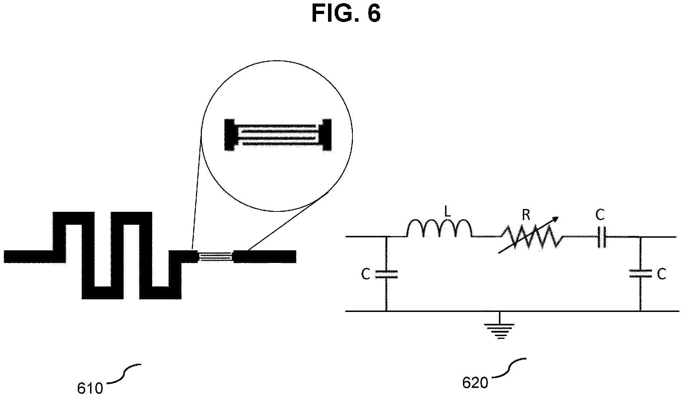

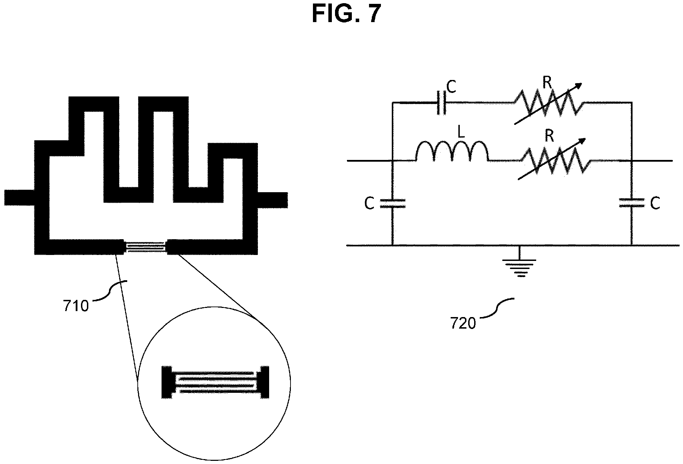

**FIG. 6** depicts an aspect 610 in which an LC resonant circuit is used for sensing, composed of lumped element inductive and capacitive components in series configuration. The right-hand figure is the respective circuit model 620. The resonance can be in series or parallel. In another aspect 710 of **FIG. 7****,** a series of resonant circuits and a parallel resonant circuit are shown with their respective circuit models 720. Meander and twisted transmission lines form the inductive component for circuit miniaturization. The capacitor is formed using interdigital fingers on the transmission line, as depicted in the amplified section. With resonant circuits, resonant shifts and resonator quality factor variations are used for sensing. The use of meander inductors and interdigitated capacitors allows the sensor to have a resonant frequency response. The presence of gas modifies the value of permittivity or resistivity which modifies the resonance and quality factor used for sensing, respectively.

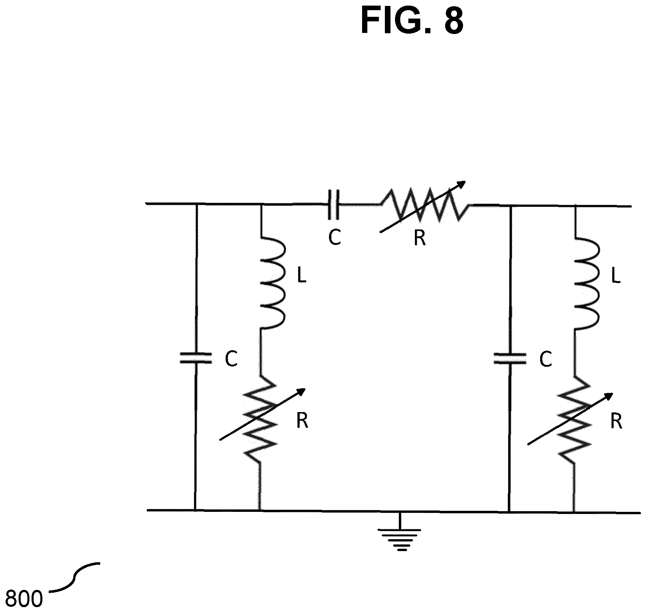

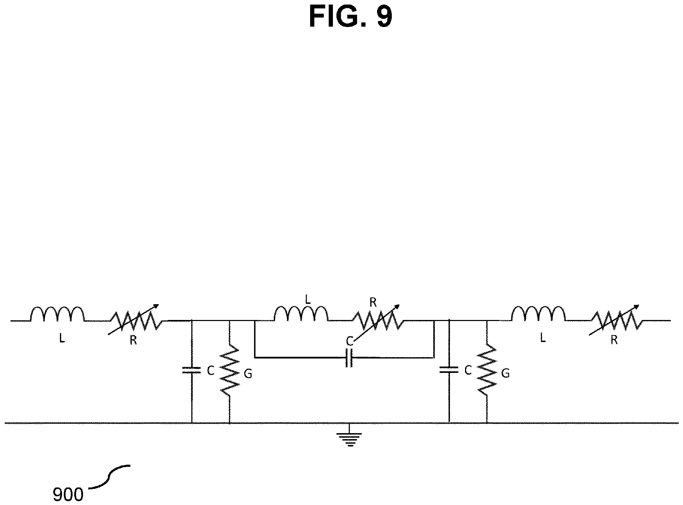

In another aspect, alternative resonant circuits for sensing include coupled resonator topologies as shown in the two models of **FIG. 8** and **FIG. 9,** where the value of R corresponds to the resistance change in the selective metal or metal oxide, the circuit models can be implemented in lumped or distributed form for a given design. A meander inductor in the middle of a transmission line is used for sensing.

The sensor in **FIG. 8** is obtained by connecting inductors and capacitors in PI configuration as shown in the model 800. The model also includes the conductivity of the gas sensitive metal represented as resistive elements.

By inserting an inductor in the middle of a transmission line we obtain a sensor as shown in **FIG. 9.** The presence of the gas not only modifies the resistivity/conductivity of the transmission line but also modifies the resistivity/conductivity of the inductor. The sensitive metal conductivity is included in the model 900 as resistance for both the line and the inductor. In all example implementations which comprise a resonant circuit, such as in FIGs. 6 to 9, material permittivity changes allow to identify the frequency shifts.

Modulating the sensitivity of the sensor is important as it needs to be adapted to the type of gas it is sensing as well as the sensing environment. Therefore, the thickness of the at least one active layer of the sensor is modulated to change the sensor's sensitivity. In one aspect, as the thickness is increased, the sensitivity of the sensor is also increased.

For the case of transmission lines, the length of the active layer is configured to increase as a function of the increase in thickness of the active layer, or the length of the active layer is configured to decrease as a function of the decrease in thickness of the active layer. In FIGs. 4 and 5, gas sensing is performed by computing the overall radio frequency signal loss transmitted through the transmission line, therefore, longer transmission lines result in a higher loss change. There is a compromise between transmission line length and transmission line and active layer thickness, these two parameters are optimized for maximum sensing performance. The longer the transmission line, the thicker the active layer should be to obtain maximum sensing performance without signal degradation.

On the other hand, for the case of resonant circuits, the sensor is configured with a thicker active layer to obtain narrow resonances with maximum sensitivity, or the sensor is configured with a thinner active layer to obtain wider resonances with lower sensitivity. In FIGs. 6 to 9, gas sensing is performed using resonant circuits and by computing the resonant frequency, phase and quality factor shifts. When using resonant circuits, narrow resonances involve high sensitivity devices, however the transmission line and active layer thickness requirements are thicker compared to wide resonances. Wide resonances can be used for gas detection with lower cost, thinner transmission lines and active layers.

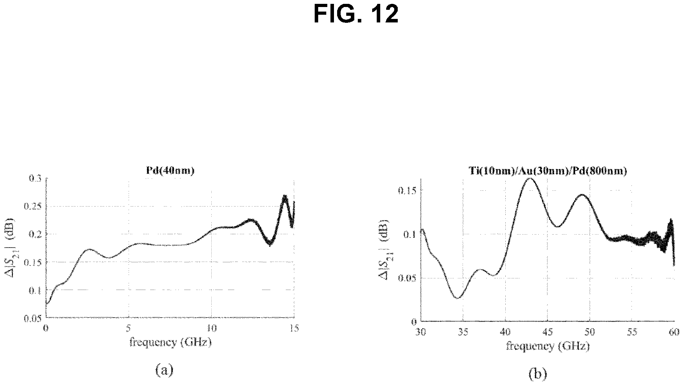

**FIG. 12** shows one set of exemplary experimental results corresponding to the aspect of FIG. 4. To demonstrate that the palladium layer of the sensor tag changes conductivity due to the presence of DMMP due to hydrogen bonds on the palladium layer, the difference in the transmission parameter ΔS₂₁ is tracked. Two initial designs were tested, consisting of short, 25 mm length straight transmission lines, one with only a thin layer of palladium at low frequencies up to a 15 GHz, and one with a stack of titanium, gold, and palladium at frequencies from 30 to 60 GHz. The result is summarized for ΔS₂₁, where a smaller change of 0.16 dB was observed for the stacked layer configuration when compared to the thin palladium film and it confirms that up to 0.25 dB of difference is obtained. No information is available in the literature for palladium conductivity change characterized at microwave frequencies, hence these results are very interesting, as they prove, on one hand, the use of palladium advantageously for gas detection, and, on the other hand, that it can be implemented as a thin film.

Implementation of passive inductively coupled radio frequency identification, RFID, sensors opens numerous opportunities where the high quality of sensor performance is needed at low-cost and when battery-free operation is critical. The proposed sensor tags are planar and easy to conform to different surfaces, including wearable sensors. The wireless sensor tags are made with batch semiconductor fabrication techniques, making it adequate for mass production with low-cost. The fabrication techniques used include photolithography, metal evaporation, atomic layer deposition, lift-off and dicing to define the sensor tags.

Hence, different aspects of the invention present a different way of detecting nerve agents using low-cost wireless sensor tags that can conform to different surfaces and can be easily integrated on unmanned vehicles or used as wearable sensor tags. The proposed low-cost wireless sensor tags can conform easily to different surfaces and sensor data can be displayed on a handheld receiver for stand-off detection. Since microwave circuits and signals have not been explored before for nerve agent detection, the sensing technique and phenomena will open up new applications in wireless sensor networks for nerve agent detection.

## Claims

1. A sensor (200; 300) for the detection of gases by microwaves, the sensor comprising a sensor module comprised of a substrate (110) and at least one active layer (210; 310) configured to allow microwave frequency signal flow, wherein the at least one active layer is a layer of a metal oxide of thickness ranging between 10 nm to 3 µm which exhibits a property variation when in contact with the gas and gas detection is determined as the microwave frequency signal flowing through the sensor is affected by the property variation, wherein the sensitivity of the sensor module is a function of the thickness of the at least one active layer.

2. The sensor of claim 1, wherein the gas is a nerve agent gas, such as sarin, or a chemically equivalent compound, such as dimethyl methylphosphonate DMMP.

3. The sensor of claim 2, wherein the property variation comprises a change in resistivity, conductivity, permittivity, or any combination thereof.

4. The sensor of claim 3, wherein the microwave frequency signal is fed to the at least one active layer as an electrical signal, or wherein the microwave frequency signal is induced in the at least one active layer once subjected to radio frequency wave irradiation.

5. The sensor of claim 4, wherein the layers of the sensor are configured to operate as an RF transmission line (510), capacitor (610), resistor (410), LC resonant circuit (610; 710; 800; 900), or any combination thereof (710).

6. The sensor of claim 5, wherein as the thickness of the at least one active layer is increased, the sensitivity of the sensor is increased.

7. The sensor of claim 6, wherein, for the case of transmission lines, the length of the active layer is configured to increase as a function of the increase in thickness of the active layer, or the length of the active layer is configured to decrease as a function of the decrease in thickness of the active layer.

8. The sensor of claim 6, wherein, for the case of resonant circuits, the sensor is configured with a thicker active layer to obtain narrow resonances with maximum sensitivity, or the sensor is configured with a thinner active layer to obtain wider resonances with lower sensitivity.

9. The sensor of claim 4, wherein the material of the substrate is microwave laminate, glass, quartz, or flexible plastic material.

10. The sensor of claim 1, wherein the metal oxide is one of zinc oxide ZnO, titanium oxide TiO₂, tin oxide SnO₂, copper oxide CuO, or tungsten oxide WO₃.

11. The sensor of claim 10, further comprising a layer of titanium (Ti), or chromium (Cr), deposited in-between the active layer and the substrate.

12. The sensor of claim 11, further comprising a layer of gold (Au), or silver (Ag), deposited on top of the layer of titanium (Ti) or chromium (Cr).

13. The sensor of claim 12, wherein another layer of titanium (Ti), or chromium (Cr), is added in between the active sensing layer and the gold (Au) layer.

14. The sensor of claim 4, configured to operate in a microwave frequency range from sub 6 GHz up to 67 GHz, wherein the sensor further comprises a radio frequency module configured to emit the microwave frequency, either via a transmission line or as wireless irradiation.

15. The sensor of claim 4,
further comprising a processing module configured for determining the presence of the gas as a function of the property variation of the active layer, as the microwave signal flowing through the sensor is affected by the property variation; or
further comprising a communications module configured for communicating with an external electronic device.

16. A system (1000) for the detection of gases by microwaves in a contaminated area (1020), the system comprising:
a sensor (1030) according to claim 1 configured on an object (1010) to be sent into the contaminated area;
an electronic device (1040) configured to communicate with the sensor for receiving data enabling the identification of the detected gas.

17. The system of claim 16,
wherein the object is a ground vehicle or an airborne vehicle, or wherein the object is a wearable item; or
further comprising a radio frequency module configured to feed a microwave frequency signal to the sensor, or configured to emit microwave frequency waves onto the sensor; or
further comprising a processing module configured for determining the presence of the gas as a function of the property variation of the active layer, as the RF signal flowing through the sensor is affected by the property variation; or
further comprising a communications module configured for communicating with an external electronic device.

## Patentansprüche

1. Sensor (200; 300) zum Nachweis von Gasen durch Mikrowellen, wobei der Sensor ein Sensormodul umfasst, das aus einem Substrat (110) und mindestens einer aktiven Schicht (210; 310) besteht, die dazu ausgelegt ist, einen Mikrowellenfrequenzsignalfluss zu ermöglichen, wobei die mindestens eine aktive Schicht eine Schicht aus einem Metalloxid mit einer Dicke im Bereich von 10 nm bis 3 µm ist, die eine Eigenschaftsänderung zeigt, wenn sie in Kontakt mit dem Gas ist, und der Gasnachweis bestimmt wird, wenn das durch den Sensor fließende Mikrowellenfrequenzsignal durch die Eigenschaftsänderung beeinflusst wird, wobei die Empfindlichkeit des Sensormoduls eine Funktion der Dicke der mindestens einen aktiven Schicht ist.

2. Sensor nach Anspruch 1, wobei das Gas ein Nervenkampfstoffgas, wie Sarin, oder eine chemisch äquivalente Verbindung, wie Dimethylmethylphosphonat DMMP, ist.

3. Sensor nach Anspruch 2, wobei die Eigenschaftsänderung eine Änderung des spezifischen Widerstands, der Leitfähigkeit, der Dielektrizitätskonstante oder eine beliebige Kombination davon umfasst.

4. Sensor nach Anspruch 3, wobei das Mikrowellenfrequenzsignal der mindestens einen aktiven Schicht als elektrisches Signal zugeführt wird, oder wobei das Mikrowellenfrequenzsignal in der mindestens einen aktiven Schicht induziert wird, sobald diese einer Hochfrequenzwellenbestrahlung ausgesetzt ist.

5. Sensor nach Anspruch 4, wobei die Schichten des Sensors dazu ausgelegt sind, als HF-Übertragungsleitung (510), Kondensator (610), Widerstand (410), LC-Resonanzkreis (610; 710; 800; 900) oder eine beliebige Kombination davon (710) zu arbeiten.

6. Sensor nach Anspruch 5, wobei mit zunehmender Dicke der mindestens einen aktiven Schicht die Empfindlichkeit des Sensors zunimmt.

7. Sensor nach Anspruch 6, wobei im Fall von Übertragungsleitungen die Länge der aktiven Schicht dazu ausgelegt ist, als Funktion der Zunahme der Dicke der aktiven Schicht zuzunehmen, oder die Länge der aktiven Schicht dazu ausgelegt ist, als Funktion der Abnahme der Dicke der aktiven Schicht abzunehmen.

8. Sensor nach Anspruch 6, wobei im Fall von Resonanzkreisen der Sensor mit einer dickeren aktiven Schicht ausgelegt ist, um enge Resonanzen mit maximaler Empfindlichkeit zu erhalten, oder der Sensor mit einer dünneren aktiven Schicht ausgelegt ist, um breitere Resonanzen mit geringerer Empfindlichkeit zu erhalten.

9. Sensor nach Anspruch 4, wobei das Material des Substrats ein Mikrowellenlaminat, Glas, Quarz oder ein flexibles Kunststoffmaterial ist.

10. Sensor nach Anspruch 1, wobei das Metalloxid eines der folgenden ist: Zinkoxid ZnO, Titanoxid TiO₂, Zinnoxid SnO₂, Kupferoxid CuO oder Wolframoxid WO₃.

11. Sensor nach Anspruch 10, der ferner eine Schicht aus Titan (Ti) oder Chrom (Cr) umfasst, die zwischen der aktiven Schicht und dem Substrat abgeschieden ist.

12. Sensor nach Anspruch 11, der ferner eine Schicht aus Gold (Au) oder Silber (Ag) umfasst, die auf der Schicht aus Titan (Ti) oder Chrom (Cr) abgeschieden ist.

13. Sensor nach Anspruch 12, wobei zwischen der aktiven Sensorschicht und der Goldschicht (Au-Schicht) eine weitere Schicht aus Titan (Ti) oder Chrom (Cr) hinzugefügt wird.

14. Sensor nach Anspruch 4, der für den Betrieb in einem Mikrowellenfrequenzbereich von unter 6 GHz bis 67 GHz ausgelegt ist, wobei der Sensor ferner ein Hochfrequenzmodul umfasst, das für die Emission der Mikrowellenfrequenz entweder über eine Übertragungsleitung oder als drahtlose Bestrahlung ausgelegt ist.

15. Sensor nach Anspruch 4,
ferner umfassend ein Verarbeitungsmodul, das zum Bestimmen des Vorhandenseins des Gases als Funktion der Eigenschaftsänderung der aktiven Schicht ausgelegt ist, da das durch den Sensor fließende Mikrowellensignal durch die Eigenschaftsänderung beeinflusst wird; oder
ferner umfassend ein Kommunikationsmodul, das für die Kommunikation mit einer externen elektronischen Vorrichtung ausgelegt ist.

16. System (1000) zum Nachweis von Gasen durch Mikrowellen in einem kontaminierten Bereich (1020), wobei das System umfasst:
einen Sensor (1030) nach Anspruch 1, der an einem Objekt (1010) ausgelegt ist, das in den kontaminierten Bereich geschickt werden soll;
eine elektronische Vorrichtung (1040), die für die Kommunikation mit dem Sensor zum Empfangen von Daten ausgelegt ist, die die Identifizierung des nachgewiesenen Gases ermöglichen.

17. System nach Anspruch 16,
wobei das Objekt ein Bodenfahrzeug oder ein Luftfahrzeug ist oder wobei das Objekt ein tragbarer Gegenstand ist; oder
ferner umfassend ein Hochfrequenzmodul, das dazu ausgelegt ist, dem Sensor ein Mikrowellenfrequenzsignal zuzuführen, oder dazu ausgelegt ist, Mikrowellenfrequenzwellen auf den Sensor auszusenden; oder
ferner umfassend ein Verarbeitungsmodul, das zum Bestimmen des Vorhandenseins des Gases als Funktion der Eigenschaftsänderung der aktiven Schicht ausgelegt ist, da das durch den Sensor fließende HF-Signal durch die Eigenschaftsänderung beeinflusst wird; oder
ferner umfassend ein Kommunikationsmodul, das für die Kommunikation mit einer externen elektronischen Vorrichtung ausgelegt ist.

## Revendications

1. Capteur (200 ; 300) de détection de gaz par micro-ondes, le capteur comprenant un module de capteur constitué d'un substrat (110) et d'au moins une couche active (210 ; 310) conçue pour permettre le flux de signaux micro-ondes, dans lequel l'au moins une couche active est une couche d'un oxyde métallique dont l'épaisseur est comprise entre 10 nm et 3 µm qui présente une modification de propriété lorsqu'il est en contact avec le gaz et la détection de gaz est déterminée lorsque le signal micro-onde circulant dans le capteur est influencé par la modification de propriété, dans lequel la sensibilité du module de capteur varie fonction de l'épaisseur de l'au moins une couche active.

2. Capteur selon la revendication 1, dans lequel le gaz est un gaz neurotoxique, tel que le sarin, ou un composé chimiquement équivalent, tel que le méthylphosphonate de diméthyle DMMP.

3. Capteur selon la revendication 2, dans lequel la modification de propriété comprend un changement de résistivité, de conductivité, de permittivité ou leur quelconque combinaison.

4. Capteur selon la revendication 3, dans lequel le signal micro-onde est envoyé à l'au moins une couche active sous forme de signal électrique, ou dans lequel le signal de fréquence micro-onde est induit dans l'au moins une couche active une fois soumis à un rayonnement d'ondes radiofréquences.

5. Capteur selon la revendication 4, dans lequel les couches du capteur sont conçues pour fonctionner comme une ligne de transmission RF (510), un condensateur (610), une résistance (410), un circuit résonant LC (610 ; 710 ; 800 ; 900), ou leur quelconque combinaison (710).

6. Capteur selon la revendication 5, dans lequel à mesure que l'épaisseur de l'au moins une couche active augmente, la sensibilité du capteur augmente.

7. Capteur selon la revendication 6, dans lequel, en cas de lignes de transmission, la longueur de la couche active augmente en fonction de l'augmentation de l'épaisseur de la couche active, ou la longueur de la couche active diminue en fonction de la diminution de l'épaisseur de la couche active.

8. Capteur selon la revendication 6, dans lequel, en cas de circuits résonants, le capteur est conçu avec une couche active plus épaisse pour obtenir des résonances étroites avec une sensibilité maximale, ou le capteur est conçu avec une couche active plus mince pour obtenir des résonances plus larges avec une sensibilité plus faible.

9. Capteur selon la revendication 4, dans lequel le matériau du substrat est un stratifié micro-ondes, du verre, du quartz ou un matériau plastique flexible.

10. Capteur selon la revendication 1, dans lequel l'oxyde métallique est un oxyde parmi l'oxyde de zinc ZnO, l'oxyde de titane TiO₂, l'oxyde d'étain SnO₂, l'oxyde de cuivre CuO ou l'oxyde de tungstène WO₃.

11. Capteur selon la revendication 10, comprenant en outre une couche de titane (Ti) ou de chrome (Cr), déposée entre la couche active et le substrat.

12. Capteur selon la revendication 11, comprenant en outre une couche d'or (Au) ou d'argent (Ag), déposée sur la couche de titane (Ti) ou de chrome (Cr).

13. Capteur selon la revendication 12, dans lequel une autre couche de titane (Ti) ou de chrome (Cr) est ajoutée entre la couche de détection active et la couche d'or (Au).

14. Capteur selon la revendication 4, conçu pour fonctionner dans une plage de fréquences micro-ondes allant de sous 6 GHz à 67 GHz, le capteur comprenant en outre un module radiofréquences conçu pour émettre la fréquence micro-ondes, soit par l'intermédiaire d'une ligne de transmission, soit sous forme de rayonnement sans fil.

15. Capteur selon la revendication 4,
comprenant en outre un module de traitement conçu pour déterminer la présence du gaz en fonction de la modification de propriété de la couche active, lorsque le signal micro-onde circulant dans le capteur est influencé par la modification de propriété ; ou
comprenant en outre un module de communications conçu pour communiquer avec un dispositif électronique externe.

16. Système (1000) de détection de gaz par micro-ondes dans une zone contaminée (1020), le système comprenant :
un capteur (1030) selon la revendication 1 conçu sur un objet (1010) à envoyer dans la zone contaminée ;
un dispositif électronique (1040) configuré pour communiquer avec le capteur pour recevoir des données permettant d'identifier le gaz détecté.

17. Système selon la revendication 16,
dans lequel l'objet est un véhicule terrestre ou un véhicule aérien, ou dans lequel l'objet est un objet portable ; ou
comprenant en outre un module de radiofréquence conçu pour injecter un signal micro-onde dans le capteur, ou conçu pour émettre des ondes micro-ondes sur le capteur ; ou
comprenant en outre un module de traitement conçu pour déterminer la présence du gaz en fonction de la modification de propriété de la couche active, lorsque le signal **RF** circulant dans le capteur est influencé par la modification de propriété ; ou
comprenant en outre un module de communications conçu pour communiquer avec un dispositif électronique externe.
